# EUROPEAN PATENT APPLICATION

(11) **EP 4 737 555 A1**
(43) Date of publication of application: **06.05.2026**
(21) Application number: 24831710.9
(22) Date of filing: 14.06.2024
(51) Int. Cl.: C12M 3/00, C12N 5/071

(54) **LIQUID-FILLED CULTURE BAG, METHOD FOR PRODUCING LIQUID-FILLED CULTURE BAG, AND METHOD FOR USING LIQUID-FILLED CULTURE BAG**

(30) Priority: 27.06.2023 JP 2023105009
(71) Applicant: Toyo Seikan Group Holdings, Ltd., Shinagawa-ku Tokyo 141-8627 (JP)
(72) Inventor: TANAKA Satoshi, Yokohama-shi, Kanagawa 240-0062 (JP)
(74) Representative: Hasegawa, Kan
(86) International application number: PCT/JP2024/021622
(87) International publication number: WO 2025/004845

(57) **Abstract**

Provided is a liquid-filled culture bag capable of efficiently removing air bubbles accumulated in minute wells in the culture bag without requiring a lot of labor or dedicated equipment, and capable of reducing troublesome work and the risk of contamination at a production site. A liquid-filled culture bag in which a culture bag having a microstructure formed in at least a part of a culture portion is housed in an outer packaging portion, wherein a space capable of being depressurized is present between an outside of the culture portion and the outer packaging portion, and the culture bag is vacuum-packaged. It is preferable that a plurality of first protrusions be provided outside the culture portion. In addition, it is preferable that a plurality of second protrusions be provided in a region of the outer packaging portion facing the outside of the culture portion. Furthermore, it is preferable that a spacer portion that forms a space capable of being depressurized be provided between the outside of the culture portion and the outer packaging portion.

## Description

### Technical Field

The present invention relates to a cell culture technique, and particularly to a culture bag provided with minute wells or the like in a culture portion.

### Background Art

When spheroids or organoids of adherent cells, such as iPS cells or ES cells, are cultured, a culture bag or the like, in which a large number of minute wells are formed in a culture portion, may be used. When such a culture bag is filled with a culture medium, air bubbles are trapped and accumulated in the minute wells, which causes a problem that interferes with cell culture. That is, when air bubbles remain in the minute wells of the culture bag, there is a problem that the formation of spheres (cell aggregates) composed of iPS cells and the like is inhibited, but it is extremely difficult to remove all the air bubbles from the minute wells.

### Citation List

### Patent Document

Patent Document 1: WO 2021/241665 A
Patent Document 2: JP 2020-80670 A

### Summary of Invention

### Technical Problem

As a means for removing the air bubbles accumulated in the minute wells, there is a method for removing the air bubbles by water pressure by spraying a liquid onto the air bubbles using, for example, a micropipette. In such an operation using a micropipette, however, there is a case where the air bubbles cannot be sufficiently removed particularly when the wells are small.

In addition, this method is applicable only to an open container, and is not suitable for removing the air bubbles accumulated in the culture portion of the culture bag.

Here, Patent Document 1 discloses that a culture bag having minute wells in a culture portion is filled with a culture medium, and then pressure is applied to the culture bag to remove air from the inside of the culture bag.

In addition, Patent Document 2 discloses that air bubbles accumulated in minute wells are removed by filling a culture bag having minute wells in a culture portion with a degassed culture medium filling liquid. Furthermore, this document also discloses that air is removed from the inside of a culture bag by disposing the culture bag having minute wells in a culture portion in a vacuum exhaust device and exposing the culture bag to a vacuum.

However, these methods have a problem that it takes a lot of labor to remove air bubbles and a problem that dedicated equipment is required. In addition, in the regenerative medicine and the production of cellular pharmaceuticals, an adjustment operation for removing air bubbles from a culture bag is required on site, and thus there is a problem in that the production process or the like becomes complicated and contamination may occur.

Therefore, the present inventors have made intensive studies and developed a liquid-filled culture bag capable of solving these problems, thereby completing the present invention.

That is, the present invention has been made in view of the above circumstances, and an object thereof is to provide a liquid-filled culture bag, a method for producing a liquid-filled culture bag, and a method for using a liquid-filled culture bag, which can efficiently remove air bubbles accumulated in minute wells in the culture bag without requiring a lot of labor or dedicated equipment, and can reduce troublesome work and the risk of contamination at a production site.

### Solution to Problem

In order to achieve the above object, a liquid-filled culture bag of the present invention is a liquid-filled culture bag in which a culture bag having a microstructure formed in at least a part of a culture portion is housed in an outer packaging portion, and has a configuration in which a space capable of being depressurized is present between the outside of the culture portion and the outer packaging portion, and the culture bag is vacuum-packaged.

In addition, the liquid-filled culture bag of the present invention is preferably configured to include a plurality of first protrusions outside the culture portion, is also preferably configured to include a plurality of second protrusions in a region of the outer packaging portion facing the outside of the culture portion, and is also preferably configured to include a spacer portion that forms a space capable of being depressurized between the outside of the culture portion and the outer packaging portion.

In addition, it is preferable that the liquid-filled culture bag of the present invention has a configuration in which the microstructure is a plurality of wells or a plurality of grooves.

In addition, it is preferable that the liquid-filled culture bag of the present invention has a configuration in which the oxygen permeability of a member in a region provided with the culture portion in the culture bag is 3000 ml/m²·day·atm or more.

In addition, the liquid-filled culture bag of the present invention is preferably configured such that a member in the region of the outer packaging portion facing the outside of the culture portion has an oxygen permeability equal to or less than the oxygen permeability of the culture portion, and more preferably configured such that the member in the region of the outer packaging portion facing the outside of the culture portion has a gas barrier property with an oxygen permeability of 1000 ml/m²·day·atm or less.

Furthermore, it is also preferable that the liquid-filled culture bag of the present invention has a configuration in which the above-described liquid-filled culture bags are variously combined.

A method for producing a liquid-filled culture bag of the present invention is a method for producing a liquid-filled culture bag in which a culture bag having a microstructure formed in at least a part of a culture portion is housed in an outer packaging portion, a space capable of being depressurized being present between the outside of the culture portion and the outer packaging portion, the method including: filling the culture bag with a liquid; and vacuum-packaging the culture bag.

In addition, it is preferable that the method for producing a liquid-filled culture bag of the present invention be made a method in which a plurality of first protrusions are provided outside the culture portion, and/or a plurality of second protrusions are provided in a region of the outer packaging portion facing the outside of the culture portion, and/or a spacer portion that forms a space capable of being depressurized is provided between the outside of the culture portion and the outer packaging portion.

A method for using a liquid-filled culture bag of the present invention is a method for using the above-described liquid-filled culture bag, the method including storing or transporting the liquid-filled culture bag at normal temperature, under refrigeration, or in a frozen state.

The method for using a liquid-filled culture bag of the present invention is a method for using the above-described liquid-filled culture bag, the method including injecting a cell, a culture medium, and cytokines, such as a cell adhesion factor and a cell growth factor, into the liquid-filled culture bag to perform culture.

### Advantageous Effects of Invention

According to the present invention, it is possible to provide a liquid-filled culture bag, a method for producing a liquid-filled culture bag, and a method for using a liquid-filled culture bag, which can efficiently remove air bubbles accumulated in minute wells in a culture bag without requiring a lot of labor or dedicated equipment, and can reduce troublesome work and the risk of contamination at a production site.

### Brief Description of Drawings

FIG. 1 is a schematic view illustrating a configuration of a liquid-filled culture bag according to an embodiment of the present invention.
FIG. 2 is a schematic cross-sectional view illustrating a configuration of a liquid-filled culture bag according to an embodiment of the present invention.
FIG. 3 is a schematic cross-sectional view illustrating a configuration of Modified Example 1 of a liquid-filled culture bag according to an embodiment of the present invention.
FIG. 4 is a schematic cross-sectional view illustrating a configuration of Modified Example 2 of a liquid-filled culture bag according to an embodiment of the present invention.
FIG. 5 is a schematic cross-sectional view illustrating a configuration of Modified Example 3 of a liquid-filled culture bag according to an embodiment of the present invention.
FIG. 6 is photographs showing the results of Test 1.
FIG. 7 is photographs showing the results of Test 2.
FIG. 8 is photographs showing the results of Test 3.
FIG. 9 is photographs showing the results of Test 4.

### Description of Embodiments

Hereinafter, embodiments of a liquid-filled culture bag, a method for producing a liquid-filled culture bag, and a method for using a liquid-filled culture bag of the present invention will be described in detail. However, the present invention is not limited to the specific contents of the following embodiments and examples.

First, a liquid-filled culture bag according to an embodiment of the present invention will be described in detail with reference to the drawings.

The liquid-filled culture bag of the present embodiment is a liquid-filled culture bag in which a culture bag having a microstructure formed in at least a part of a culture portion is housed in an outer packaging portion, and is characterized in that a space capable of being depressurized is present between the outside of the culture portion and the outer packaging portion, and the culture bag is vacuum-packaged.

That is, in the liquid-filled culture bag, the culture bag having a microstructure formed in at least a part of the culture portion is filled with a liquid, and the culture bag is housed in the outer packaging portion.

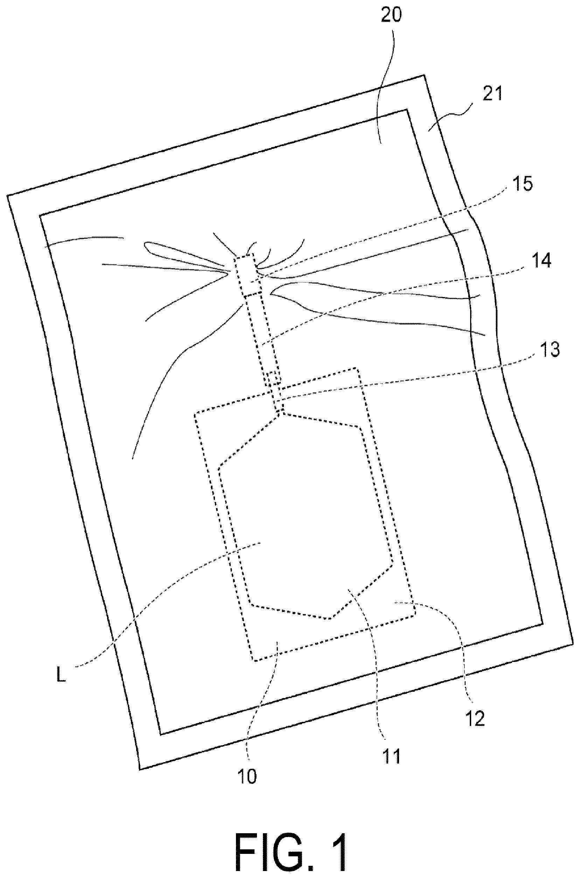

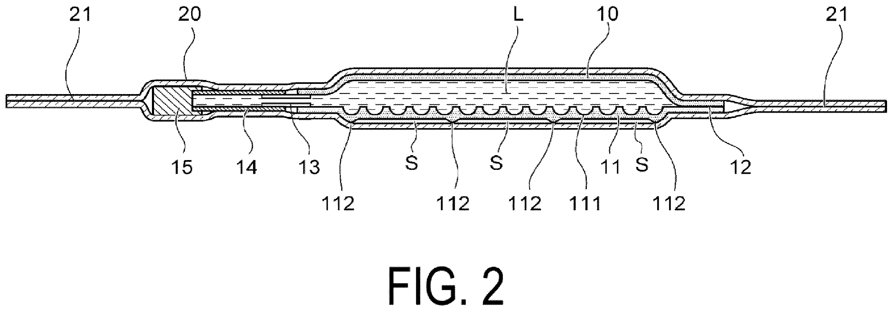

Specifically, the liquid-filled culture bag of the present embodiment includes a culture bag 10 and an outer packaging portion 20, and the culture bag 10 is sealed in the outer packaging portion 20, as illustrated in FIGS. 1 and 2.

The culture bag 10 is a bag-shaped container made of a soft packaging material for culturing cells and the like, and includes a culture portion 11 having a culture surface that is a region for culturing cells therein.

The outside of the culture portion 11 is the opposite side (the side on which first protrusions 112 are provided in FIG. 2) of a culture surface in a bag wall portion (the bottom surface portion of the bag in FIG. 2) provided with the culture portion 11. That is, the outside of the culture portion 11 is a region corresponding to the culture surface on the outside of the culture bag 10.

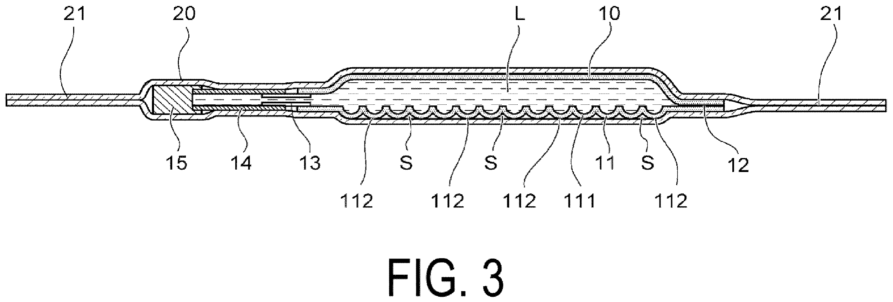

Although the culture portion 11 is provided only on the bottom surface side of the culture bag 10 in FIG. 2, the culture portion 11 may be provided only on the top plate side of the culture bag 10, or the culture portion 11 may be provided on both the bottom surface side and the top plate side. Also in these cases, the outside of the culture portion 11 on the top plate side is a region corresponding to the culture surface on the top plate side on the outside of the culture bag 10, and the outside of the culture portion 11 on the bottom surface side is a region corresponding to the culture surface on the bottom surface side on the outside of the culture bag 10. The same applies to the modified examples illustrated in FIGS. 3 to 5.

The culture bag 10 can be produced by, for example, sandwiching one or more ports between two films and heat-sealing the peripheral edge portion. In FIG. 1, the culture bag 10 includes a bag sealing portion 12 formed by heat-sealing, one port 13, a tube 14 communicating with the port 13, and a cap 15 for sealing the tube 14. Note that the method for producing the culture bag 10 is not limited thereto, and the culture bag 10 can also be produced by other methods, for example, by using a vacuum pressure-formed film or an inflation-molded film.

In addition, the number of ports provided in the culture bag 10 is not limited to one, but may be two or more.

Furthermore, the sealing of the tube 14 is not limited to the method using the cap 15, but for example, the tube 14 may be sealed using a connector having a cap function, or a general-purpose connector may be connected to the tube 14 and a cap for sealing the connector may be provided.

A microstructure 111 is formed in the culture portion 11 of the culture bag 10. The microstructure 111 may be, for example, a plurality of wells or a plurality of grooves. In addition, it is also possible to perform a low cell-adhesion treatment, a cell-adhesion treatment, or the like on the culture portion 11 having the microstructure 111.

The shape of the plurality of wells is not particularly limited, but may be, for example, a hemispherical shape, a conical shape, a pyramidal shape, a shape formed of a cylinder and a cone, or a shape formed of a prism and a pyramid. In addition, the shape of the plurality of grooves is also not particularly limited, but may be, for example, a shape in which the cross section perpendicular to the longitudinal direction of the grooves is V-shaped and linear or curved.

The shape of the opening in the plurality of wells is preferably a circle, a square, or the like, and the diameter of the circle or inscribed circle can be, for example, 2 mm or less, may be 1.5 mm or less, or may be 1.0 mm or less.

The depth of the plurality of wells can be, for example, 1.5 mm or less, may be 1.0 mm or less, may be 0.5 mm or less, or may be 0.35 mm or less.

Furthermore, each of the width and depth of the plurality of grooves can similarly be, for example, 1.5 mm or less, may be 1.0 mm or less, may be 0.5 mm or less, or may be 0.35 mm or less.

By forming the microstructure 111 in the culture portion 11 into a plurality of wells, it is possible to suitably form spheres including a plurality of iPS cells, other adherent cells, and the like using the culture bag 10.

In addition, by forming the microstructure 111 in the culture portion 11 into a plurality of grooves, it is possible to suitably culture the adherent cells by adhering the adherent cells to the culture portion 11 in a state where the area of the culture surface of the culture bag 10 is increased.

The culture bag 10 is filled with a liquid L. As the liquid L, a culture medium, water for injection, a phosphate buffer solution, physiological saline, or the like is preferably used.

In the liquid-filled culture bag of the present embodiment, when the air bubbles are once removed from the microstructure 111 in the culture portion 11 and the surface of the microstructure 111 is wetted, air bubbles are not usually caused again in the microstructure 111. Therefore, it is also preferable to fill with not only a culture medium but also other liquids, such as water for injection, as the liquid L.

In the culture bag 10, it is preferable that at least a member in a region provided with the culture portion 11 be made of a gas permeable member so that air bubbles accumulated in the microstructure 111 can be removed to the outside of the culture bag 10. For example, the oxygen permeability of the member in the region of the culture portion 11 at 37°C is preferably 3000 ml/m²·day·atm or more, more preferably 5000 ml/m²·day·atm or more, and still more preferably 8000 ml/m²·day·atm or more.

In the liquid-filled culture bag of the present embodiment, a space S capable of being depressurized (depressurized space) is present between the outside of the culture portion 11 and the outer packaging portion 20.

As described above, the outside of the culture portion 11 is a region corresponding to the culture surface on the outside of the culture bag 10.

As illustrated in FIG. 2, the liquid-filled culture bag of the present embodiment can be configured such that the depressurized space S is suitably formed between the outside of the culture portion 11 and the outer packaging portion 20 by providing a plurality of the first protrusions 112 on the outside of the culture portion 11.

In addition, it is also preferable that the liquid-filled culture bag of the present embodiment have a shape in which the bottom surface portion of the culture bag 10 is a continuous body having a U-shape and both the wells (microstructure 111) inside the bag and the protrusions (first protrusions 112) outside the bag are provided, as illustrated in Modified Example 1 of FIG. 3.

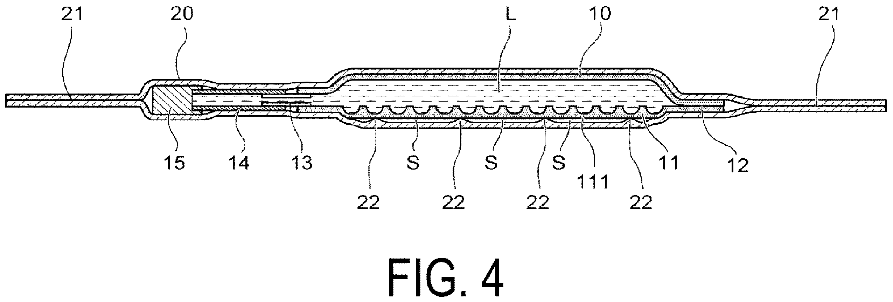

Furthermore, the liquid-filled culture bag of the present embodiment can also be configured such that the depressurized space S is suitably formed between the outside of the culture portion 11 and the outer packaging portion 20 by providing a plurality of second protrusions 22 in a region of the outer packaging portion 20 facing the outside of the culture portion 11, as illustrated in Modified Example 2 of FIG. 4.

The first protrusions 112 and the second protrusions 22 may be any protrusions as long as the depressurized space S can be formed between the outside of the culture portion 11 and the outer packaging portion 20, and the shape, size, and number thereof are not particularly limited.

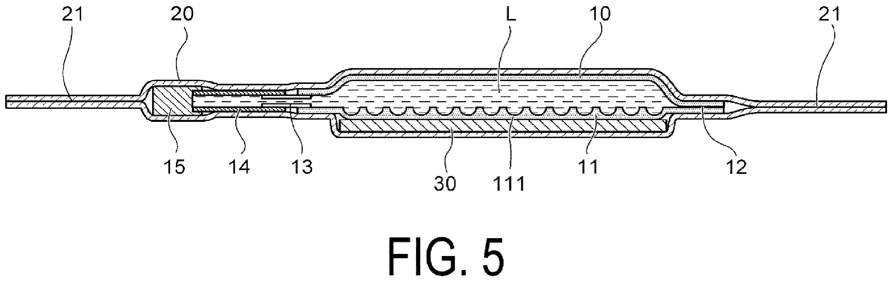

Furthermore, the liquid-filled culture bag of the present embodiment can also be configured such that the depressurized space S is suitably formed between the outside of the culture portion 11 and the outer packaging portion 20 by providing a spacer portion 30 that forms a space capable of being depressurized between the outside of the culture portion 11 and the outer packaging portion 20, as illustrated in Modified Example 3 of FIG. 5.

The spacer portion 30 may be any spacer portion as long as a space capable of being depressurized can be formed between the outside of the culture portion 11 and the outer packaging portion 20, and the specific structure thereof is not particularly limited, but for example, a plate-shaped member having a plurality of through-holes or a porous member can be suitably used.

Note that the liquid-filled culture bag of the present embodiment may be one in which the culture bag 10 is double-packaged by sealing the culture bag 10 in another outer packaging portion and by sealing another outer packaging portion in the outer packaging portion 20.

Even if the liquid-filled culture bag of the present embodiment is configured in this manner, for example, in a case where another outer packaging portion functions as the above-described spacer portion 30 and thus the depressurized space S is formed between the outside of the culture portion 11 and the outer packaging portion 20, it is possible to suitably remove air bubbles from the culture portion 11.

Furthermore, in the liquid-filled culture bag of the present embodiment, it is also preferable to provide a plurality of the first protrusions 112 on the outside of the culture portion 11 and to provide a plurality of the second protrusions 22 in a region of the outer packaging portion 20 facing the outside of the culture portion 11. It is also preferable to provide a plurality of the first protrusions 112 on the outside of the culture portion 11 and to provide the spacer portion 30 that forms a space capable of being depressurized between the outside of the culture portion 11 and the outer packaging portion 20. It is also preferable to provide a plurality of the second protrusions 22 in a region of the outer packaging portion 20 facing the outside of the culture portion 11 and to provide the spacer portion 30 that forms a space capable of being depressurized between the outside of the culture portion 11 and the outer packaging portion 20. It is also preferable to provide all of these configurations at the same time.

According to the liquid-filled culture bag of the present embodiment, by forming such a depressurized space S, the outside of the culture portion 11 and the outer packaging portion 20 are not completely adhered to each other in the end, and a space in a vacuum or depressurized state is generated in the outer packaging portion 20, so that it is possible to efficiently remove the air bubbles accumulated in the microstructure 111 to the depressurized space S.

The liquid-filled culture bag of the present embodiment is vacuum-packaged in a state where the culture bag 10 is sealed in the outer packaging portion 20.

That is, in the liquid-filled culture bag of the present embodiment, the inside of the outer packaging portion 20 is in a vacuum or depressurized state, and is in a substantially vacuum state.

This vacuum-packaging can be performed using a commercially available heat-sealing device or the like. For example, an industrial vacuum-sealer available from Fujiimpulse Co., Ltd. can be suitably used.

In the liquid-filled culture bag of the present embodiment, an outer sealing portion 21 is formed in the peripheral edge portion of the outer packaging portion 20.

In the liquid-filled culture bag of the present embodiment, the outer packaging portion 20 may not have a gas barrier property, but preferably has a gas barrier property.

As will be described later in Examples, it is because, in a case where a material having a gas barrier property is used for the outer packaging portion 20, the time for removing air bubbles accumulated in the microstructure 111 can be shortened more than in a case where a material having no gas barrier property is used.

Specifically, in the liquid-filled culture bag of the present embodiment, the oxygen permeability of the member in the region of the outer packaging portion 20 facing the outside of the culture portion 11 is preferably equal to or less than the oxygen permeability of the culture portion 11, more preferably 8000 ml/m²·day·atm or less, still more preferably 1000 ml/m²·day·atm or less, and particularly preferably 100 ml/m²·day·atm or less.

Here, in Test 3 described below, it is shown that even if the oxygen permeability of the film in the culture portion is equal to the oxygen permeability of the outer packaging portion, it is possible to remove air bubbles from the culture portion although it takes time. In addition, as the oxygen permeability of the outer packaging portion is set to be equal to or less than the oxygen permeability of the film in the culture portion and the difference therebetween is increased, the amount of gas that exits from the culture portion side to the depressurized space S becomes larger than the amount of gas that enters the depressurized space S from the outside of the outer packaging portion. Therefore, it is possible to shorten the time for removing the air bubbles accumulated in the culture portion.

In the liquid-filled culture bag of the present embodiment, a resin film or the like can be suitably used as the material of the culture bag 10, and a polyolefin-based resin, such as polyethylene, polypropylene, or polymethylpentene, can be used. Examples thereof include polyethylene, a copolymer of ethylene and α-olefin, a copolymer of ethylene and vinyl acetate, and an ionomer using a copolymer of ethylene and acrylic acid or methacrylic acid, and a metal ion. In addition, a styrene-based elastomer, a polyester-based thermoplastic elastomer, or the like can also be used. Furthermore, soft vinyl chloride resin, polybutadiene resin, chlorinated polyethylene resin, polyurethane-based thermoplastic elastomer, silicon-based thermoplastic elastomer, styrene-based elastomer such as SBS (styrene-butadiene-styrene), SIS (styreneisoprene-styrene), SEBS (styrene-ethylene-butylene-styrene), or SEPS (styrene-ethylenepropylene-styrene), fluorine-based resin, or the like may be used.

In the liquid-filled culture bag of the present embodiment, as the material of the outer packaging portion 20, for example, polyethylene, polypropylene, a laminated film having a sealant layer and a barrier layer, or a vapor deposition film (examples of the barrier layer include ethylene-vinyl alcohol copolymer (EVOH), nylon, or polyamide; examples of vapor deposition include aluminum vapor deposition, alumina vapor deposition, silica vapor deposition, or the like) can be used.

According to such a liquid-filled culture bag of the present embodiment, it is possible to efficiently remove air bubbles accumulated in the minute wells in the culture bag without requiring a lot of labor or dedicated equipment, and it is possible to reduce troublesome work and the risk of contamination at a production site.

A method for producing a liquid-filled culture bag of the present embodiment is a method for producing a liquid-filled culture bag in which a culture bag having a microstructure formed in at least a part of a culture portion is housed in an outer packaging portion, a space capable of being depressurized being present between the outside of the culture portion and the outer packaging portion, the method being characterized by including: filling the culture bag with a liquid; and vacuum-packaging the culture bag.

In addition, it is preferable that the method for producing a liquid-filled culture bag of the present embodiment be made a method in which a plurality of first protrusions are provided outside the culture portion, and/or a plurality of second protrusions are provided in a region of the outer packaging portion facing the outside of the culture portion, and/or a spacer portion that forms a space capable of being depressurized is provided between the outside of the culture portion and the outer packaging portion.

According to such a method for producing a liquid-filled culture bag of the present embodiment, air bubbles present in the microstructure formed in the culture portion of the culture bag can be removed to the space between the outside of the culture portion and the outer packaging portion, and it is possible to obtain a liquid-filled culture bag in which air bubbles are removed from the microstructure.

In addition, it is also possible to check liquid leakage from the culture bag by providing a space capable of being depressurized between the outside of the culture portion and the outer packaging portion and vacuum-packaging the culture bag in a state where the culture bag is filled with a liquid.

A method for using a liquid-filled culture bag of the present embodiment is a method for using the above-described liquid-filled culture bag of the present embodiment, the method being characterized by including storing or transporting the liquid-filled culture bag at normal temperature, under refrigeration, or in a frozen state.

In addition, it is also preferable that the method for using a liquid-filled culture bag of the present embodiment be made a method including injecting target cells, a culture medium, and cytokines, such as a cell adhesion factor and a cell growth factor, into the above-described liquid-filled culture bag of the present embodiment to perform culture.

Furthermore, it is also preferable that the method be made a method including injecting, after the pre-filled liquid is once removed from the culture bag, target cells and culture media to perform culture.

According to such a method for using a liquid-filled culture bag of the present embodiment, the liquid-filled culture bag can be stored or transported in a state where the culture bag is filled with a liquid, and it is possible to remove air bubbles during storage and transportation.

In the liquid-filled culture bag of the present embodiment in such a manner of use, air bubbles are removed in advance, and thus it is possible to suitably perform cell culture without performing the work of removing air bubbles from the wells prior to the cell culture.

### Examples

Hereinafter, tests performed to confirm the effects of removing air bubbles by the liquid-filled culture bag, the method for producing a liquid-filled culture bag, and the method for using a liquid-filled culture bag according to the embodiment of the present invention will be described.

### (Test 1)

A test was performed to confirm that a liquid-filled culture bag, from which air bubbles are removed, can be obtained by the present embodiment.

Specifically, the culture bag illustrated in FIGS. 1 and 3 was first produced. A polyethylene film (available from Toyo Seikan Group Holdings, Ltd.) was used as a material of the culture bag, and two films of 100 mm × 70 mm × 0.10 mm were prepared.

In addition, a plurality of hemispherical wells were formed in the culture portion of the culture bag. The diameters of the openings of the wells were 0.5 mm and the depths thereof were 0.25 mm, and about 18000 wells were formed.

On the outside of the culture portion of the culture bag, protrusions, each having a hemispherical shape, a height of 250 µm, and an interval of about 500 µm, were formed. The oxygen permeability of the film of the culture bag was 8,500 ml/m²·day·atm (37°C - 50% RH).

Then, the film on which the culture surface was formed and the other film were bonded by heat-sealing. At this time, one port was formed, and a needleless connector (available from Qosina) was connected to the port via a tube.

Next, the produced culture bag was filled with 15mL of a culture medium (StemFit AK02N (Ajinomoto Co., Inc., product number RCAK02N)), and only large air bubbles were removed by sucking out with a syringe.

Then, the culture bag filled with the culture medium was sealed in an outer packaging portion (Copack (R) ST1525, Asahi Kasei Packs Co., Ltd.), and was vacuum-packaged using a commercially available vacuum-sealer (V301, Fujiimpulse Co., Ltd.), thereby producing the liquid-filled culture bag of the present embodiment.

The film of the outer packaging portion had a structure of ONy #15/LDPE20/L-LDPE #40 (total thickness: 75 µm), and the oxygen permeability thereof was 23 ml/m²·day·atm (23°C - 50% RH).

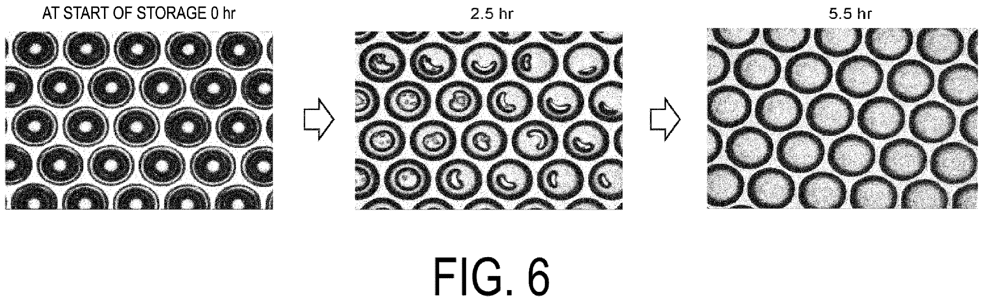

Next, the obtained liquid-filled culture bag was stored in a refrigerator at 4°C, and micrographs of the wells in the culture surface of the liquid-filled culture bag were taken at the time of the start of storage, after 2.5 hours, and after 5.5 hours. The result is shown in FIG. 6.

A state is shown in the photograph at the time of the start of storage (0 hr) in FIG. 6, in which air bubbles are accumulated in the black circles in the wells. Note that the white circle at the center is due to reflection of light, but air bubbles are present in this area.

Next, substantially crescent-shaped particles are shown in the wells in the photograph after 2.5 hours (2.5 hr) in FIG. 6, but it is seen that the particles are air bubbles that have become small and the air bubbles are partially removed. Note that external water droplets caused by condensation are reflected in this photograph.

Furthermore, no black circle is shown in the wells in the photograph after 5.5 hours (5.5 hr) in FIG. 6, and it is observed that air bubbles are completely removed.

From this result, it was found that, according to the present embodiment, a liquid-filled culture bag from which air bubbles are removed is suitably obtained.

### (Test 2)

A test was performed to confirm that cell culture can be performed using the liquid-filled culture bag obtained by the present embodiment.

Specifically, the liquid-filled culture bag produced in Test 1 was stored for 3 months and used to perform cell culture.

All the culture medium was discharged from the liquid-filled culture bag stored for 3 months, the culture bag was filled with 20 ml of StemFit AK02N (Ajinomoto Co., Inc., product number RCAK02N) containing 10 mM Y-27632 (Wako Pure Chemical Corporation), and human induced pluripotent stem (iPS) cells (1231A3 strain) were seeded so that about 150 cells were housed in each well.

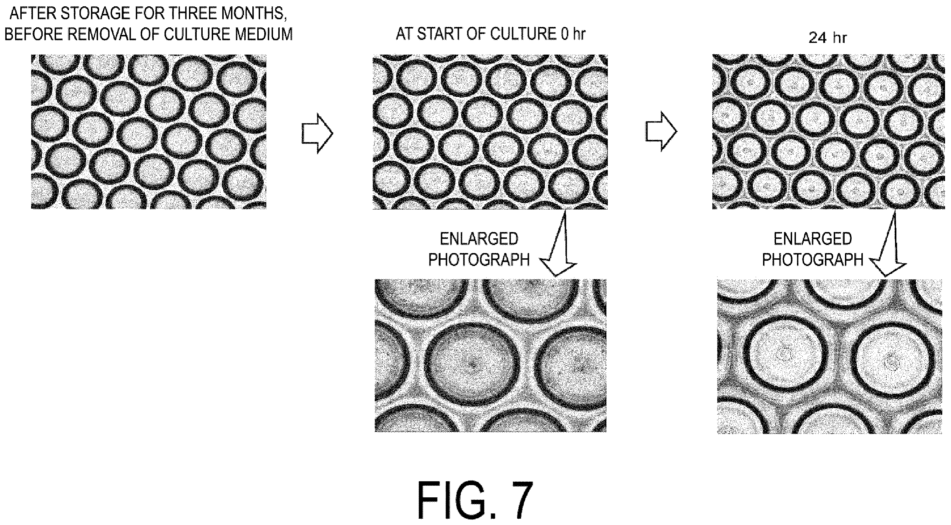

Then, micrographs of the wells in the culture surface of the liquid-filled culture bag were taken 24 hours after the start of culture. The result is shown in FIG. 7.

The photograph at the left end in FIG. 7 shows the state of the culture surface before the culture medium is removed from the liquid-filled culture bag stored for 3 months. As shown in this photograph, no air bubbles were present in the wells in the culture surface.

The upper and lower photographs at the center in FIG. 7 show the state of the culture surface at the time of the start of culture (0 hr), and a plurality of small particles in the wells in the lower enlarged photograph are cells.

The upper and lower photographs at the right end in FIG. 7 show the state of the culture surface 24 hours after the start of culture (24 hr), and it is observed that spheroids are formed at the center of each well.

From this result, it was clarified that, according to the liquid-filled culture bag of the present embodiment, air bubbles are removed in advance, and thus it is possible to suitably perform cell culture without performing the work of removing air bubbles from the wells prior to the cell culture.

### (Test 3)

A test was performed to confirm whether, in a case where a gas permeable material was used as the material of the outer packaging portion of the liquid-filled culture bag of the present embodiment, air bubbles are removed.

Specifically, the same culture bag as in Test 1 was first produced. A polyethylene film (available from Toyo Seikan Group Holdings, Ltd.) was used as a material of the culture bag, and two films of 100 mm × 70 mm × 0.10 mm were prepared.

In addition, a plurality of hemispherical wells were formed in the culture surface of the culture bag. The diameters of the openings of the wells were 0.5 mm and the depths thereof were 0.25 mm, and about 18000 wells were formed.

On the outside of the culture portion of the culture bag, protrusions, each having a hemispherical shape, a height of 250 µm, and an interval of about 500 µm, were formed. The oxygen permeability of the film of the culture bag was 8,500 ml/m²·day·atm (37°C - 50% RH).

Then, the film on which the culture surface was formed and the other film were bonded by heat-sealing. At this time, one port was formed, and a needleless connector (available from Qosina) was connected to the port via a tube.

Next, the produced culture bag was filled with 15mL of a culture medium (StemFit AK02N (Ajinomoto Co., Inc., product number RCAK02N)), and only large air bubbles were removed by sucking out with a syringe.

Then, the culture bag filled with the culture medium was sealed in an outer packaging portion having a gas permeability, and was vacuum-packaged using a commercially available vacuum-sealer (V301, Fujiimpulse Co., Ltd.), thereby producing the liquid-filled culture bag of the present embodiment. As the outer packaging portion, a bag made by heat-sealing a polyethylene film (available from Toyo Seikan Group Holdings, Ltd.), which was identical to that used for the culture bag, into the same size as the Copack (R) ST1525 used in Test 1 was used. The film of the outer packaging portion had an oxygen permeability of 8,500 ml/m²·day·atm (37°C - 50% RH).

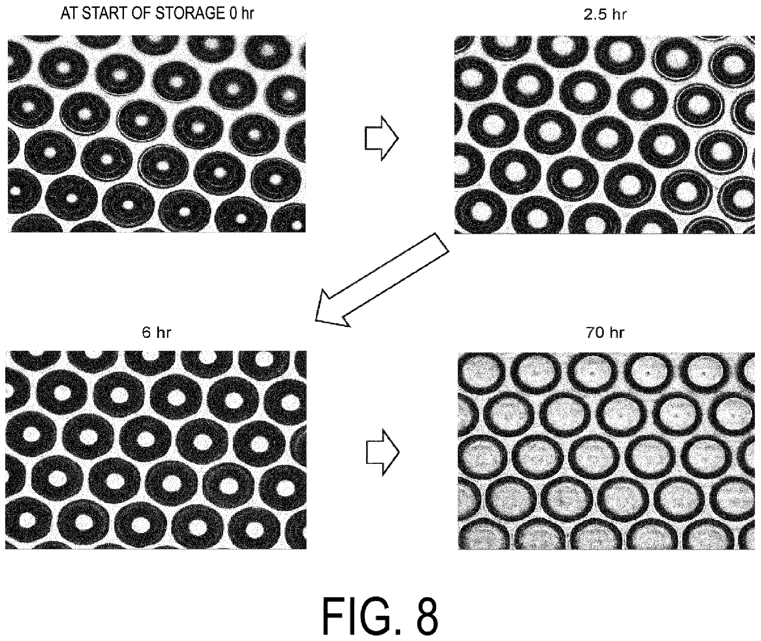

Next, the obtained liquid-filled culture bag was stored in a refrigerator at 4°C, and micrographs of the wells in the culture surface of the liquid-filled culture bag were taken at the time of the start of storage, after 2.5 hours, after 6 hours, and after 70 hours. The result is shown in FIG. 8.

A state is shown in the photograph at the time of the start of storage (0 hr) in FIG. 8, in which air bubbles are accumulated in the black circles in the wells.

In addition, states are shown in the photographs after 2.5 hours (2.5 hr) and after 6 hours (6 hr) in FIG. 8, in which air bubbles are still accumulated in the black circles in the wells.

On the other hand, no black circle is shown in the wells in the photograph after 70 hours (70 hr) in FIG. 8, and it is observed that air bubbles are completely removed.

From this result, it was confirmed that, in a case where a material having a gas permeability is used as the material of the outer packaging portion of the liquid-filled culture bag of the present embodiment, air bubbles are removed, although it takes a longer time to remove the air bubbles than in a case where a material having a gas barrier property is used.

### (Test 4)

A test was performed to confirm whether or not, in a case where a culture bag was stored without using the liquid-filled culture bag of the present embodiment, a liquid-filled culture bag from which air bubbles were removed was able to be obtained.

Specifically, the same culture bag as in Test 1 was first produced. A polyethylene film (available from Toyo Seikan Group Holdings, Ltd.) was used as a material of the culture bag, and two films of 100 mm × 70 mm × 0.10 mm were prepared.

A plurality of hemispherical wells were formed as a microstructure in the culture surface of the culture bag. The diameters of the openings of the wells were 0.5 mm and the depths thereof were 0.25 mm, and about 18000 wells were formed.

On the outside of the culture portion of the culture bag, protrusions, each having a hemispherical shape, a height of 250 µm, and an interval of about 500 µm, were formed. The oxygen permeability of the film of the culture bag was 8,500 ml/m²·day·atm (37°C - 50% RH).

Then, the film on which the culture surface was formed and the other film were bonded by heat-sealing. At this time, one port was formed, and a needleless connector (available from Qosina) was connected to the port via a tube.

Next, the produced culture bag was filled with 15mL of a culture medium (StemFit AK02N (Ajinomoto Co., Inc., product number RCAK02N)), and only large air bubbles were removed by sucking out with a syringe.

Then, the culture bag filled with the culture medium was sealed in an outer packaging portion (Copack (R) ST1525, Asahi Kasei Packs Co., Ltd.), and was packaged without being vacuumed, using a commercially available vacuum-sealer (V301, Fujiimpulse Co., Ltd.), thereby producing a liquid-filled culture bag of a comparative example.

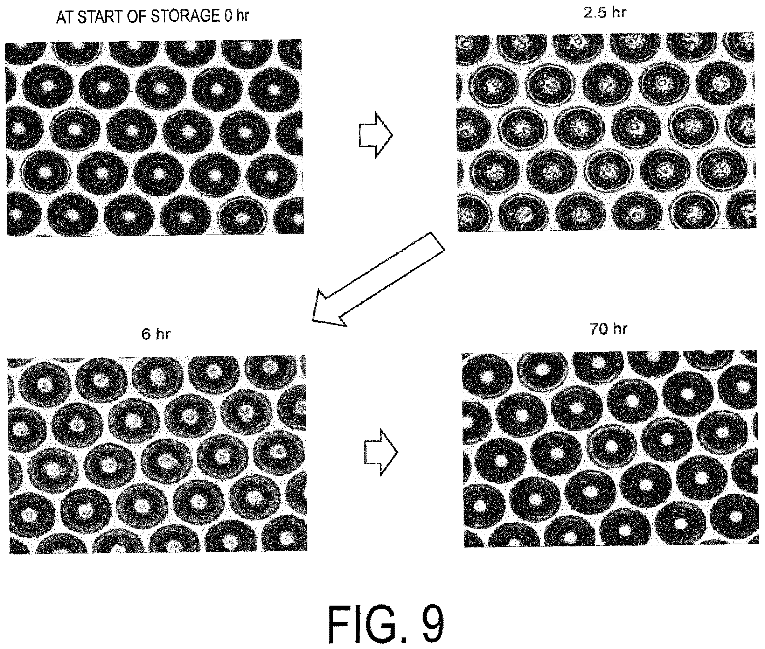

Next, the obtained liquid-filled culture bag of the comparative example was stored in a refrigerator at 4°C, and micrographs of the wells in the culture surface of the liquid-filled culture bag were taken at the time of the start of storage, after 2.5 hours, after 6 hours, and after 70 hours. The result is shown in FIG. 9.

A state is shown in the photograph at the time of the start of storage (0 hr) in FIG. 9, in which air bubbles are accumulated in the black circles in the wells.

In addition, a state is shown in the photographs after 2.5 hours (2.5 hr) in FIG. 9, in which air bubbles are also accumulated in the black circles in the wells.

Furthermore, states are shown in the photographs after 6 hours (6 hr) and after 70 hours (70 hr) in FIG. 9, in which air bubbles are also accumulated in the black circles in the wells.

As described above, it was found that air bubbles in the microstructure formed in the culture portion of a culture bag cannot be removed only by packaging and storing the culture bag without providing a space capable of being depressurized between the outside of the culture portion of the culture bag and the outer packaging portion.

The present invention is not limited only to the embodiments and examples described above, and it goes without saying that various modifications can be made within the scope of the present invention.

For example, it is possible to appropriately change the method by selecting a culture bag having a size capable of forming, for example, 500000 to 1000000 spheres and sealing the culture bag in the outer packaging portion to produce a liquid-filled culture bag.

### Industrial Applicability

The present invention can be suitably used in a case where a liquid-filled culture bag, filled with a culture medium or the like in advance to remove air bubbles from a culture portion, is used.

The contents of the documents described in this description and the Japanese application of the present application are all incorporated herein.

### Reference Signs List

10 Culture bag
11 Culture portion
111 Microstructure
112 First protrusion
12 Bag sealing portion
13 Port
14 Tube
15 Cap
20 Outer packaging portion
21 Outer sealing portion
22 Second protrusion
30 Spacer portion
S Depressurized space
L Liquid

## Claims

1. A liquid-filled culture bag in which a culture bag having a microstructure formed in at least a part of a culture portion is housed in an outer packaging portion, wherein a space capable of being depressurized is present between an outside of the culture portion and the outer packaging portion, and the culture bag is vacuum-packaged.

2. The liquid-filled culture bag according to claim 1, wherein a plurality of first protrusions are provided outside the culture portion.

3. The liquid-filled culture bag according to claim 1 or 2, wherein a plurality of second protrusions are provided in a region of the outer packaging portion facing the outside of the culture portion.

4. The liquid-filled culture bag according to claim 1 or 2, comprising a spacer portion that forms a space capable of being depressurized between the outside of the culture portion and the outer packaging portion.

5. The liquid-filled culture bag according to claim 1 or 2, wherein the microstructure is a plurality of wells or a plurality of grooves.

6. The liquid-filled culture bag according to claim 1 or 2, wherein an oxygen permeability of a member in a region provided with the culture portion in the culture bag is 3000 ml/m²·day·atm or more.

7. The liquid-filled culture bag according to claim 1 or 2, wherein an oxygen permeability of a member in the region of the outer packaging portion facing the outside of the culture portion is equal to or less than the oxygen permeability of the culture portion.

8. The liquid-filled culture bag according to claim 7, wherein the member in the region of the outer packaging portion facing the outside of the culture portion has a gas barrier property with an oxygen permeability of 1000 ml/m²·day·atm or less.

9. A method for producing a liquid-filled culture bag in which a culture bag having a microstructure formed in at least a part of a culture portion is housed in an outer packaging portion, a space capable of being depressurized being present between an outside of the culture portion and the outer packaging portion, the method comprising:
filling the culture bag with a liquid; and
vacuum-packaging the culture bag.

10. The method for producing a liquid-filled culture bag according to claim 9, wherein
a plurality of first protrusions are provided outside the culture portion, and/or a plurality of second protrusions are provided in a region of the outer packaging portion facing the outside of the culture portion, and/or a spacer portion that forms a space capable of being depressurized is provided between the outside of the culture portion and the outer packaging portion.

11. A method for using the liquid-filled culture bag described in claim 1 or 2, the method comprising storing or transporting the liquid-filled culture bag at normal temperature, under refrigeration, or in a frozen state.

12. A method for using the liquid-filled culture bag according to claim 1 or 2, the method comprising injecting a cell, a culture medium, and cytokines, such as a cell adhesion factor and a cell growth factor, into the liquid-filled culture bag to perform culture.
